# EUROPÄISCHE PATENTSCHRIFT

(11) **EP 1 441 673 B1**
(45) Veröffentlichungstag und Bekanntmachung des Hinweises auf die Patenterteilung: **31.12.2008**
(21) Anmeldenummer: 01980097.8
(22) Anmeldetag: 07.11.2001
(51) Int. Cl.: A61F 2/34, A61F 2/30, B21D 28/02, A61F 2/46

(54) **VERFAHREN ZUR HERSTELLUNG EINER KÜNSTLICHEN GELENKPROTHESE MIT DECKEL**
METHOD OF PRODUCTION OF AN ARTIFICIAL JOINT PROSTHESIS WITH CLOSING ELEMENT
PROCEDE DE PRODUCTION D'UNE PROTHESE D'ARTICULATION ARTIFICIELLE AVEC ELEMENT DE FERMETURE

(43) Veröffentlichungstag der Anmeldung: 04.08.2004
(73) Patentinhaber: Kuoni, Xaver, CH-8952 Schlieren (CH)
(72) Erfinder: Kuoni, Xaver, CH-8952 Schlieren (CH)
(74) Vertreter: Schneider Feldmann AG Patent- und Markenanwälte
(86) Internationale Anmeldenummer: PCT/CH2001/000652
(87) Internationale Veröffentlichungsnummer: WO 2003/039410

(56) Entgegenhaltungen:
- EP-A- 0 601 224
- EP-A- 1 016 385
- EP-A- 1 163 891
- WO-A-96/03096
- WO-A-96/23457
- WO-A-97/33525
- DE-A- 4 334 417
- US-A- 5 935 174
- US-A- 6 120 546
- US-B1- 6 231 612

## Beschreibung

Die vorliegende Erfindung betrifft ein Verfahren zur Herstellung einer Gelenkprothese mit einem pfannenförmigen Grundkörper.

Künstliche Hüftgelenke umfassen üblicherweise eine Gelenkpfanne mit einem Einsatz, in welchen der Kopf eines Schafts eingreift. In der Praxis sind zwei Typen von Pfannen sehr gebräuchlich: konisch geformte Pfannen und sphärische Pfannen. Die Entscheidung für den einen oder den anderen Pfannentyp erfolgt je nach Indikation und Vorliebe des behandelnden Arztes.

Beide Pfannentypen umfassen jeweils eine metallische Aussenschale und einen in der Schale angeordneten Einsatz oder Inlay, der üblicherweise aus Keramik, Kunststoffen wie zum Beispiel Polyäthylen oder Metall hergestellt ist. Solche künstliche Gelenkpfannen haben sich aufgrund ihrer Konstruktion aus Schale plus Einsatz als sehr verlässlich erwiesen. Um soll mittel- bis langfristig eine optimale Sekundärstabilisierung sicherzustellen werden die Pfannen zum Beispiel mit einer rauhen Oberflächenbeschichtung versehen. Oft werden die Pfannen auch mit Spongiosa hinterfüllt, falls das vorgefräste Bett im Knochen nicht in der ganzen Tiefe von der Pfanne ausgefüllt wird. Das Hinterfüllen erfolgt nach dem Einsetzen der Pfanne und erfordert daher eine entsprechende Öffnung im Pfannenkörper. Üblicherweise ist diese Öffnung im Polbereich der Aussenschale angeordnet.

Um das anschliessend eingesetzte Inlay sicher vor einen Kontakt mit dem Knochenmaterial zu schützen , muss die polare Pfannenöffnung noch vor dem Einsetzen des Inlays dicht verschlossen werden. Ein Beispiel für ein bekanntes Verschlusssystem ist in der EP-A-0'601'224 beschrieben.

Mehrere stirnseitige Öffnungen der Pfannenschale sind durch ein auf der Innenseite der Schale gelagertes Verschlusselement verschliessbar. Die Schale weist drei etwa gleich grosse Öffnungen auf, die sich gleichmässig verteilt jeweils über ein Kreissegment von annähernd 60° erstrecken.

Das scheibenförmige Verschlusselement ist um eine Schwenkachse, welche im wesentlichen senkrecht zum stirnseitigen Bereich verläuft und mit der Rotationsachse der Pfannenschale zusammenfällt, verschwenkbar. Das Verschlusselement weist etwa gleich grosse Ausnehmungen auf, die sich gleichmässig verteilt jeweils über ein Kreissegment von annähernd 60° erstrecken. Sämtliche Öffnungen lassen sich also durch Verdrehen des Verschlusselementes vollständig verschliessen. Das Verschlusselement ist ohne Spiel und unter Vorspannung zum Beispiel mittels einer Niete an der Stirnseite befestigt.

Die US-B-6'231'612 zeigt eine kreisrunde Öffnung am Pol, welche primär zum Ansetzen des Einschlagwerkzeugs genutzt wird. Die Bohrung ist mit einem Innengewinde versehen und wird nach dem Einschlagen der Pfanne mit einem passenden Stopfen verschraubt. Im eingeschraubten Zustand Liegt der Stopfen plan zur Innenwand der Pfannenschale. Die durchgehende Oberfläche soll Mikrobewegungen und damit verbundenen Abrieb des Inlays an der Pfannnenschale vermeiden.

WO 96/23457 A1 zeigt eine Hüftgelenkpfanne, bei welchem der Hinterfüllkanal mit einem Stopfen verschlossen wird. Der becherförmige Stopfen umfasst einen Bodenteil und eine Seitenwand mit einer Abschrägung, welche sich beim Einbringen des Stopfens in den Hinterfüllkanal verformt, wodurch der Stopfen im Hinterfüllkanal festgeklemmt wird.

WO 96/03096 A1 zeigt ebenfalls eine Hüftgelenkpfanne, bei welchem der Hinterfüllkanal mit einem konkav-konvexen Deckel verschlossen wird. Der Deckel wird mit der konkaven Seite in Richtung des Knochens in die Hinterfüllöffnung eingeführt, und anschliessend durch einen Schlag auf die konvexe Seite zumindest teilweise umgestülpt, wodurch sich der verformte Deckel im Hinterfüllkanal verklemmt.

Die EP-A- 1163891 bildet einem Stand der Technik gemäss Art. 54(3) EPÜ. Dieses Dokument offenbart nicht eindeutig und zweifelsfrei, dass der Rand des Deckels eine zylindrische Mantelfläche hat (siehe den Deckel in Fig. 3 im eingebanten Zustand).

Bei den bekannten Verschlusssystemen kann nicht immer ausgeschlossen werden, dass sich das Verschlussmittel lockert oder teilweise öffnet. Geschieht dies, so kann Gewebeflüssigkeit das Inlay angreifen und mikroskopisch feine

Inlaypartikel können in das benachbarte Gewebe austreten. Die Folgen einer solchen Fehlfunktion können von Entzündungen bis hin zu langfristigen Störungen des Knochenaufbaus und des Anwachsens des Implantates im Knochen reichen. Die bekannten Verschlusssysteme sind zudem recht aufwendig herzustellen und verteuern dadurch das Implantat.

Der Erfindung liegt deshalb die Aufgabe zugrunde ein kostengünstiges Verfahren zur Herstellung einer Gelenkprothese mit einem pfannenförmigen Grundkörper und einem darin befestigbaren Deckel zu schaffen.

Diese Aufgabe wird durch das Verfahren nach 1 gelöst.

Die Erfindung umfasst die technische Lehre, dass sich ein polarer im wesentlichen zylindrischer Kanal in einer Gelenkpfannenschale mit einer leicht bombierten Scheibe verschliessen lässt, welche im Klemmsitz im Kanal gehalten ist. Die Verschlussscheibe, im folgenden auch Deckel genannt, weist einen Aussendurchmesser auf, welcher einige zehntel Millimeter bis Millimeter grösser ist als der Innendurchmesser des zu verschliessenden Kanals und wird mittels eines Einschlaginstrumentes passgenau in den Kanal eingesetzt. Weitere vorteilhafte Ausführungsvarianten ergeben sich aus der Beschreibung und den abhängigen Ansprüchen.

In den Zeichnungen sind Ausführungsbeispiele der Erfindungsgegenstände dargestellt und in der nachfolgenden Beschreibung erläutert. Es zeigt:
- Figur 1: eine erfindungsgemässe Pfannenschale mit teilweise ausgebrochener Wandung und einen zugehörigen Deckel;
- Figur 2: einen Schnitt entlang der Pfannenachse im Polbereich;

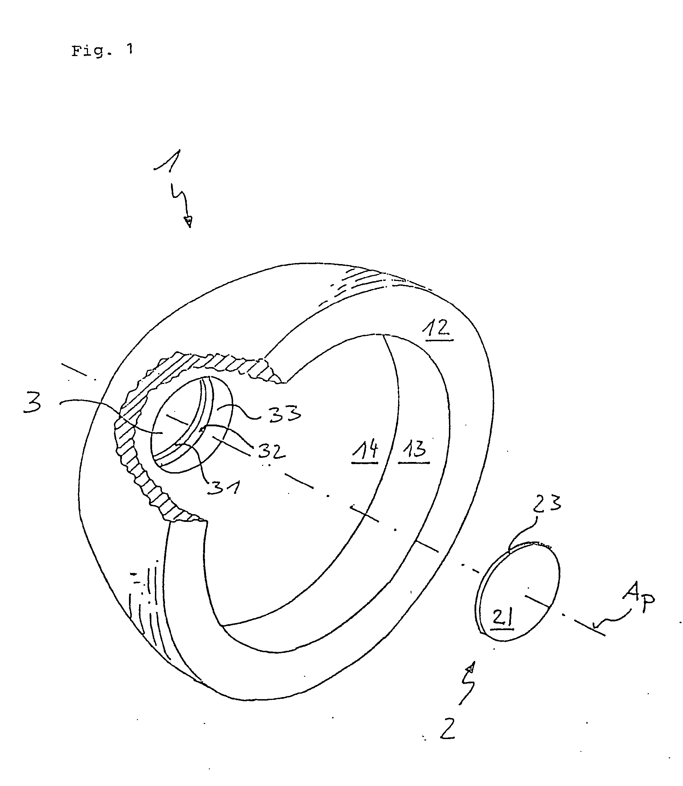

Figur 1 zeigt einen Grundkörper oder eine Schale 1 einer künstlichen Gelenkpfanne, welche bis auf die Ausgestaltung eines polaren Bereiches aus dem Stand der Technik bekannt ist, und von der Anmelderin sehr erfolgreich produziert und vertrieben wird.

Die dargestellte Pfannenschale ist am Pol leicht abgeflacht und weist also von distal, d.h. entfernt vom Pol, nach proximal, einen zunehmenden Radius auf. Bei der dargestellten Schale ist ein distaler Wandbereich weggelassen, so dass der Blick auf einen polaren Kanal 3 zum Hinterfüllen der Schale 1 von Innen her frei ist. Im vorliegenden Ausführungsbeispiel fällt die zentrale Achse des Kanals 3, bestehend aus einer zylindrischen Bohrung 33 mit einer dazu leicht verengten polaren Öffnung 30, mit der Pfannenachse A_{P} zusammen. Durch die strichlinierte Achse A_{P} soll auch angedeutet werden, wie der Deckel 2 zum Verschliessen des Kanals 3, respektive der Öffnung 30 in die Schale 1 eingeführt wird. Der Deckel 20 entspricht im wesentlichen einer leicht bombierten oder konvex gewölbten Scheibe. Der Deckel 2 ist im wesentlichen rotationssymmetrischen zur Pfannenachse (A_{P}) und weist eine konvex gekrümmten Oberseite 22 und eine konkav gekrümmte Unterseite 21 auf. Gemäß der vorliegenden Erfindung ist ein Rand der Scheibe 2 parallel zur Mittelachse ausgerichtet, so dass der Scheibenrand eine zylindrische Mantelfläche definiert. Wie im weiteren ausgeführt wird, ist diese Randgestaltung äusserst vorteilhaft für die Dichtwirkung des Deckels 2.

Der Deckel wird mit der konvexen Oberseite 22 voran in eine zylindrische Bohrung 33 eingeführt. An die zylindrische Bohrung 33 schliesst im proximalen Bereich eine umlaufende Rippe 31 an, an deren umlaufender Innenwand 32 der periphere Bereich des Deckels 2 zu liegen kommt. Die Höhe der Rippe 31 bestimmt den Durchmesser der Öffnung 30. Die Innenwand der zylindrischen Bohrung 33 ist parallel zur Pfannenlängsachse A_{P} ausgerichtet.

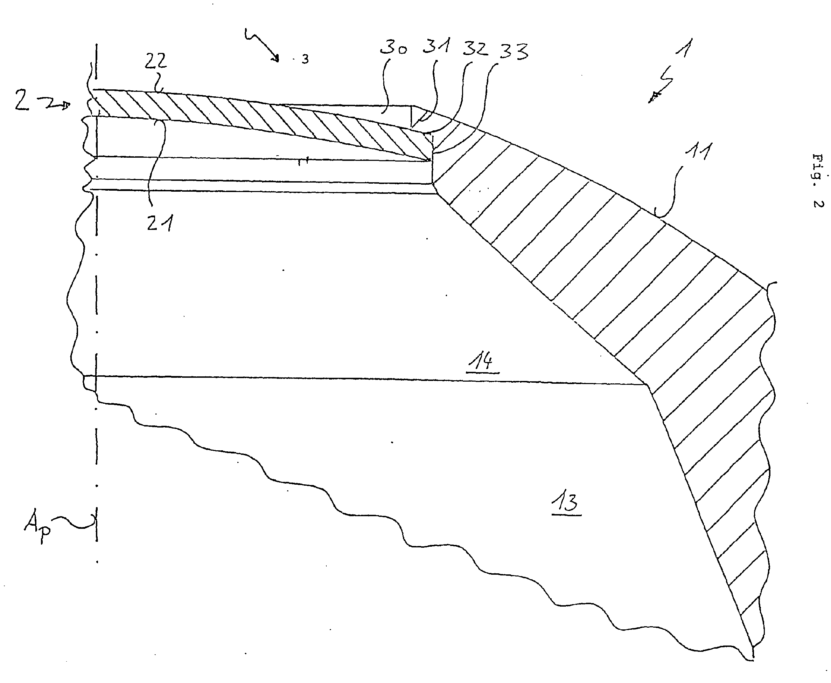

Die Verschlussscheibe 2 ist im Durchmesser einige Hundertstelmillimeter bis einige Zehntelmillimeter grösser als die zylindrische Bohrung 33, so dass ein stabiler Presssitz der eingeschlagenen Scheibe 2 gewährleistet ist. Die konvexe Gestaltung der Scheibe 2 erleichtert einerseits das Einschlagen und unterstützt andererseits den sicheren Sitz der Scheibe 2 in der zylindrischen Bohrung 33 durch ein gewisses Federmoment. Aus der Figur 2 geht hervor, dass der Rand der Scheibe 2 der Erfindung parallel zu ihrer Mittelachse ausgerichtet ist, und damit vollflächig in der zylindrischen Bohrung 33 anliegen kann.

Die Innenwand 32 der Rippe 31 ist leicht nach proximal geneigt, so dass die bombierte Scheibe 2 mit dem peripheren Bereich der Oberseite 22 annähernd vollflächig an der Rippe anliegen kann. Dadurch ist sichergestellt, dass der Deckel 2 den Schaleninnenraum zuverlässig und dauerhaft gegen das Eindringen von Flüssigkeiten oder Gewebematerial abdichtet. Andererseits ist natürlich auch das Austreten von feinsten abgeriebenen Inlay- oder Schalenpartikeln in das anliegende Gewebe unterbunden.

In einem bevorzugten Ausführungsbeispiel einer Pfanne der Grösse 56, dass heisst mit 56 mm Pfannendurchmesser, wie es in der Figur 2 dargestellt ist, ist die zylindrischen Bohrung 33 knapp unter 18 mm weit und der Durchmesser der Öffnung 30 ist 16.9 mm. Die Rippe 31 ist also annähernd 1 mm breit. Wie bereits oben beschrieben ist die Innenwand 32 der Rippe 31 um annähernd 15° nach proximal geneigt, so dass die 0.6 mm starke Scheibe 2 mit einem äusseren Krümmungsradius von etwa 46.1 mm mit dem peripheren Bereich der Oberseite 22 satt an der Rippe 31 anliegen kann. Die Vorspannung der Scheibe 2 beträgt 0.02 bis 0.5 mm, vorzugsweise 0.05 bis 0.1 mm, dass heisst, der Scheibendurchmesser ist um diesen Wert grösser als die Bohrung 33.

Da je nach Körpergrösse des Patienten verschiedene Pfannengrössen zum Einsatz kommen, hat es sich als praktisch erwiesen auch die Hinterfüllkanäle in mehreren angepassten Grössen auszugestalten. Für die Verschlussdeckel werden vorteilhafterweise drei Grössen mit folgenden Durchmessern/Krümmungsradien angeboten 15/31, 18/45 und 25/90 (alle Angaben in mm). Öffnungen unter 5 mm lassen sich nicht mehr einfach hinterfüllen und Öffnungen über 40 mm beeinträchtigen die Stabilität der Pfannenschale. Das Verhältnis von Durchmesser zum Krümmungsradius wird vorzugsweise so gewählt, dass die Höhe aller angebotenen Deckel 2 annähernd gleich ist. Es wird dadurch eine zu starke Bombierung vermieden, was dazu führen könnte, dass die Deckel über die Pfannenkontour hinaus in den zu hinterfüllenden Hohlraum ragen.

## Patentansprüche

1. Verfahren zur Herstellung einer Gelenkprothese mit einem pfannenförmigen Grundkörper (1) zum Einsetzen in einen Knochen wobei in einem Polbereich des Grundkörpers (1) mindestens ein Hinterfüllkanal (3) angeordnet ist, welcher mittels eines klemmend am Grundkörper (1) befestigbaren Deckels (2) dicht verschliessbar ist, wobei der Deckel (20) im wesentlichen rotationssymmetrisch gestaltet ist und eine konvex gekrümmte Oberseite (22) und eine konkav gekrümmte Unterseite (21) aufweist, und dass ein Deckelrand (23) parallel zur Mittelachse ausgerichtet ist, so dass der Rand (23) eine zylindrische Mantelfläche definiert und dass der pfannenförmige Grundkörper (1) im Polbereich von dem zur Pfannenachse (A_{P}) im wesentlichen rotationssymmetrischen Hinterfüllkanal (3) durchsetzt wird, welcher eine zylindrische Bohrung (33) umfasst, an welche im proximalen Bereich eine umlaufende Rippe (31) anschliesst, die eine Hinterfüllöffnung (30) definiert, wobei der Deckel (2) dessen Aussendurchmesser im 0.02 bis 0.5 mm, vorzugsweise 0.05 bis 0.1 mm grösser ist als der Innendurchmesser der zylindrischen Bohrung (33), mittels einem Einschlagwerkzeug (6) in einen stabilen Presssitz in die Bohrung (33) im Hinterfüllkanal (33) eingeschlagen wird.

2. Verfahren zur Herstellung einer Gelenkprothese nach Anspruch 1, wobei eine Innenwand (32) einer Rippe (31) nach proximal geneigt ist, und wobei der bombierte Deckel (2) mittels dem Einschlagwerkzeug (6) so weit eingeschlagen wird, dass er mit einem peripheren Bereich der konvexen Oberfläche (22) satt an der Rippe (31) zum Anliegen kommt.

## Claims

1. A process for the production of a joint prosthesis comprising a pan-shaped base body (1) for insertion into a bone, wherein at least one backfilling channel (3) is placed in a polar area of the base body (1) , which is hermetically sealable by means of a cap (2) that is affixable to the base body (1) in a clamping manner, wherein the cap (20) is substantially designed as rotationally symmetrical and comprise a convexly curved upper side (22) and a concavely curved underside (21), and that a cap edge (23) is aligned parallel to the central axis, so that the edge (23) defines a cylindrical lateral surface and that the pan-shaped base body (1) is crossed in the polar area by the backfilling channel (3), which is substantially rotationally symmetrical to the pan axis (aₚ), which encompassed a cylindrical hole (33), which is adjoined by a circumferential rib (31) which defines a backfilling opening (30), wherein the cap (2), the external diameter whereof at 0.02 to 0.5 mm, preferably 0.05 to 0.1 mm, is larger than the internal diameter of the cylindrical hole (33), is driven into the hole (33) in the backfilling channel (33) by means of an impact tool (6) into a stable interference fit.

2. The process for the production of a joint prosthesis according to claim 1, wherein an inner wall (32) of a rib (31) is tilted proximally and wherein the raised cap (2) is driven in sufficiently far by means of said impact tool (6) that it comes to abut with a peripheral area of the convex surface (22) lightly against said rib (31).

## Revendications

1. Procédé de fabrication d'une prothèse articulée comportant un corps de base (1) en forme de poche à insérer dans un os, moyennant quoi au moins un canal de remplissage arrière (3) est disposé dans une région polaire du corps de base (1), lequel peut être obturé de manière étanche au moyen d'un couvercle (2) pouvant être fixé par serrage sur le corps de base (1), moyennant quoi le couvercle (20) est configuré de manière essentiellement symétrique en rotation et présente un côté supérieur (22) incurvé de manière convexe et un côté inférieur (21) incurvé de manière concave, et en ce que un bord du couvercle (23) est aligné parallèlement à l'axe central, de telle sorte que le bord (23) définisse une surface de manteau cylindrique et en ce que le corps de base (1) en forme de poche est traversé dans la région polaire par le canal de remplissage arrière (3) essentiellement symétrique en rotation par rapport à l'axe de la poche (Ap), lequel canal comprend un alésage cylindrique (33), auquel est contiguë une nervure (31) faisant le tour de la circonférence dans la région proximale, qui définit une ouverture de remplissage arrière (30), moyennant quoi le couvercle (2), dont le diamètre extérieur est compris entre 0,02 et 0,5 mm, de préférence 0,05 et 0,1 mm, est plus grand que le diamètre intérieur de l'alésage cylindrique (33), est enfoncé au moyen d'un outil à enfoncer (6) dans une assise de pressage stable dans l'alésage (33) dans le canal de remplissage arrière (33).

2. Procédé de fabrication d'une prothèse articulée selon la revendication 1, moyennant quoi une paroi intérieure (32) d'une nervure (33) est inclinée proximalement, et moyennant quoi le couvercle bombé (2) est enfoncé au moyen de l'outil à enfoncer (6) jusqu'au point de venir reposer par une région périphérique de la surface convexe (22) à plat sur la nervure (31).
